# EUROPÄISCHE PATENTSCHRIFT

(11) **EP 1 356 836 B1**
(45) Veröffentlichungstag und Bekanntmachung des Hinweises auf die Patenterteilung: **13.11.2013**
(21) Anmeldenummer: 03006422.4
(22) Anmeldetag: 21.03.2003
(51) Int. Cl.: A61M 1/34, A61M 1/16

(54) **Vorrichtung zur extrakorporalen Blutbehandlung**
Apparatus for extracorporeal blood treatment
Appareil pour traitement extracorporel du sang

(30) Priorität: 27.04.2002 DE 10218846
(43) Veröffentlichungstag der Anmeldung: 29.10.2003
(60) Teilanmeldung: 10009424.2
(73) Patentinhaber: Fresenius Medical Care Deutschland GmbH, 61352 Bad Homburg v.d.H. (DE)
(72) Erfinder: Zhang, Wei, 97421 Schweinfurt (DE); Müller, Carsten, 97502 Euerbach (DE)
(74) Vertreter: Oppermann, Frank

(56) Entgegenhaltungen:
- DE-A- 4 239 937
- US-A1- 2001 045 395

## Beschreibung

Die Erfindung betrifft eine Vorrichtung zur Blutbehandlung mit einem extrakorporalen Blutkreislauf.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und Dialysierflüssigkeitskammer auf, die von einer semipermeablen Membran getrennt sind. Während der Behandlung strömt das Blut des Patienten durch die Blutkammer. Um das Blut effektiv von den harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinmolekularen Substanzen durch die Membran im wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei einer Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators (Prädilution) oder stromab des Dialysators (Postdilution) dem extrakorporalen Blutkreislauf zugeführt wird.
Es sind Vorrichtungen zur Hämo(dia)filtration bekannt, bei denen die Dialysierflüssigkeit online aus Frischwasser und Konzentraten und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf online aus dem Dialysierflüssigkeitssystem der Maschine über eine Substitutionsflüssigkeitsleitung zugeführt. Bei der Prädilution führt die Substitutionsflüssigkeitsleitung zu einer Anschlussstelle an der arteriellen Blutleitung stromauf des Dialysators, während bei der Postdilution die Substitutionsflüssigkeitsleitung zu einer Anschlussstelle an der venösen Blutleitung stromab des Dialysators führt.

Bei der online Blutbehandlung mit Postdilution wird das Serum im extrakorporalen Blutkreislauf über den Dialysator entzogen, so dass sich das Blut im Dialysator verdickt. Das verdickte Blut wird anschliessend durch die Zugabe der Substitutionsflüssigkeit wieder verdünnt, die stromab des Dialysators in die venöse Blutleitung strömt. Dadurch bildet sich zwischen dem Auslass des Dialysators und der Anschlussstelle der Substitutionsflüssigkeitsleitung an die venöse Blutleitung eine Flüssigkeitssäule aus verdicktem Blut. Diese Flüssigkeitssäule stellt für die Blutbehandlung grundsätzlich kein Risiko dar.

Die Erfinder haben aber erkannt, dass unter bestimmten Betriebsbedingungen der Blutbehandlungsvorrichtung die Gefahr besteht, dass verdicktes Blut mit hoher Geschwindigkeit in die Kanüle der venösen Blutleitung gelangt. Dabei kann es zu einer Gefährdung des Patienten aufgrund einer Hämolyse kommen.

Eine der Betriebsbedingungen der Blutbehandlungsvorrichtung, bei der eine Hämolyse nicht auszuschliessen ist, liegt beispielsweise dann vor, wenn die Blutbehandlung unterbrochen wird. In diesem Zusammenhang ist unter einer Unterbrechung der Blutbehandlung auch ein Aussetzen der Behandlung nur für eine sehr kurze Zeitdauer zu verstehen.

Eine Unterbrechung der Blutbehandlung ist beispielsweise dann erforderlich, wenn das Dialysierflüssigkeitssystem mittels eines konventionellen Druckhaltetests auf Undichtigkeiten überprüft wird. Beim Start des Druckhaltetests wird die Substituatpumpe abrupt gestoppt, während noch Dialysierflüssigkeit durch den Dialysator strömt. Anschliessend wird der Dialysator von dem Dialysierflüssigkeitssystem abgetrennt.

Bei den bekannten Druckhaltetests wird im Dialysierflüssigkeitssystem mittels der Ultrafiltrationspumpe ein Unterdruck erzeugt. Für den Fall, dass der Druck in einem bestimmten Zeitraum vorgegebene Druckbedingungen verfehlt, wird angenommen, dass das System eine Leckage aufweist. Andernfalls wird von der Dichtigkeit des Systems ausgegangen.

Bei der Messung des Drucks in der venösen Blutleitung haben die Erfinder positive Spitzen mit einer bestimmten Periodendauer im Drucksignal beobachtet. Es hat sich gezeigt, dass diese Periodendauer genau der Dauer einer Periode des Druckhaltetests entspricht, der zyklisch erfolgt. Die Erfinder haben erkannt, das dieses Phänomen darauf zurückzuführen ist, das beim Druckhaltetest kurzfristig verdicktes Blut in die venöse Blutleitung gelangt. Dieses Phänomen konnte auch durch einen kurzfristigen Abfall des relativen Blutvolumens nachgewiesen werden, da das Blut mit dem sehr viel höheren Hämatokrit (HKT) über den Shunt durch Rezirkulation in den arteriellen Blutschlauch zurückströmt. Es hat sich gezeigt, dass der HKT-Anstieg beim Druckhaltetest von Substituat- und Ultrafiltrationsrate abhängig ist. Eine höhere Substituatrate/Ultrafiltrationsrate begünstigt den HKT-Anstieg beim Druckhaltetest.

Eine Hämolyse ist aber auch dann nicht auszusehliessen, wenn die Blutbehandlung zwar nicht unterbrochen, aber mit geänderten Flussraten fortgeführt wird.

Der Erfindung liegt die Aufgabe zugrunde, eine Vorrichtung zur extrakorporalen Blutbehandlung anzugeben, die eine Unterbrechung oder Fortführung der Blutbehandlung mit geänderten Flussraten ohne die Gefährdung des Patienten durch Hämolyse, insbesondere bei der Überprüfung des Dialysierflüssigkeitssystems auf Undichtigkeiten mittels eines Druckhaltetests erlaubt.

Die Lösung dieser Aufgabe erfolgt erfindungsgemäss mit den Merkmalen des Patentanspruchs 1. Vorteilhafte Ausführungsformen der Erfindung sind Gegenstand der Unteransprüche.

Die erfindungsgemäß Vorrichtung beruht darauf, dass zur Unterbrechung oder Fortführung der Blutbehandlung mit geänderten Flussraten die Substituatpumpe nicht plötzlich gestoppt bzw. die Flussrate abrupt geändert wird.

Bei einer Vorrichtung, die aber nicht Gegenstand der Erfindung ist, wird die Förderrate der Substituatpumpe und die Ultrafiltrationsrate, die sich um die Nettoentzugsrate unterscheiden, mit der Flüssigkeit insgesamt dem extrakorporalen Kreislauf entzogen werden soll, von vorgegebenen Werten während vorgegebener Zeitintervalle reduziert, bevor der Dialysierflüssigkeitsfluss durch den Dialysator oder Filter (Hämofiltration) unterbrochen wird. Die Reduzierung der Förderrate der Substituatpumpe und der Ultrafiltrationsrate kann dabei kontinuierlich oder diskontinuierlich in einzelnen Schritten erfolgen. Vorzugsweise wird die Förderrate der Substituatpumpe und die Ultrafiltrationsrate im gleichen Zeitintervall reduziert. In der Praxis kann es aber ausreichend sein, wenn die Zeitintervalle, in denen die Förderrate der Substituatpumpe und die Ultrafiltrationsrate verringert werden, nicht exakt gleich sind. Die "langsame" Reduzierung der Förderrate der Substituatpumpe und der Ultrafiltrationsrate führt dazu, dass sich in der venösen Blutleitung eine genau abgegrenzte Flüssigkeitssäule aus verdicktem Blut nicht bilden kann oder zumindest derart "verschmiert" wird, dass sie keine Gefährdung mehr darstellen kann. Zur Unterbrechung der Behandlung wird die Förderrate auf Null reduziert.

Bei der erfindungsgemäßen Vorrichtung wird nach dem Reduzieren der Ultrafiltrationsrate, die Substituatpumpe mit einer vorgegebenen Förderrate noch für ein vorgegebenes Zeitintervall betrieben, bevor der
Dialysierflüssigkeitsfluss durch den Dialysator oder Filter unterbrochen wird. Die Länge des Zeitintervalls entspricht der Länge der sich in der venösen Blutleitung bildenden Flüssigkeitssäule aus verdicktem Blut.

Eine mögliche Anwendung der erfindunggsgemäßen Vorrichtung ist beispielsweise der Übergang von einer Hämodiafiltrationsbehandlung auf eine Hämodialysebehandlung. Bei der Hämodiafiltration laufen die Substituat- , Dialysierflüssigkeits- und Ultrafiltratpumpe mit vorgegebenen Förderraten.

Die Vorrichtung zur extrakorporalen Blutbehandlung gemäß der Erfindung verfügt über eine Steuereinrichtung zur Unterbrechung der Blutbehandlung, mit der die Einstellung der Förderrate der Substituatpumpe und der Ultrafiltrationsrate automatisiert ist.

Die erfindungsgemäße Vorrichtung kann in allen Fällen Anwendung finden, in denen die Blutbehandlung zu unterbrechen oder mit geänderten Flussraten fortzuführen ist, wobei unter einer Unterbrechung der Blutbehandlung auch ein nur kurzeitiges Aussetzen der Behandlung verstanden wird. Eine der Hauptanwendungsfälle sind die bekannten Verfahren zur Überwachung des Dialysierflüssigkeitssystems auf Undichtigkeiten. Diese Verfahren können von den konventionellen Druckhaltetests Gebrauch machen, bei denen im Dialysierflüssigkeitssystem ein Unterdruck erzeugt wird. Es sind aber auch alle anderen Verfahren möglich, bei denen der Dialysator oder Filter vom Dialysierflüssigkeitssystem abgetrennt wird. Beispielsweise kann die Integrität des System mit dem aus der DE 42 39 937 A1 bekannten Verfahren bzw. der Vorrichtung überprüft werden.

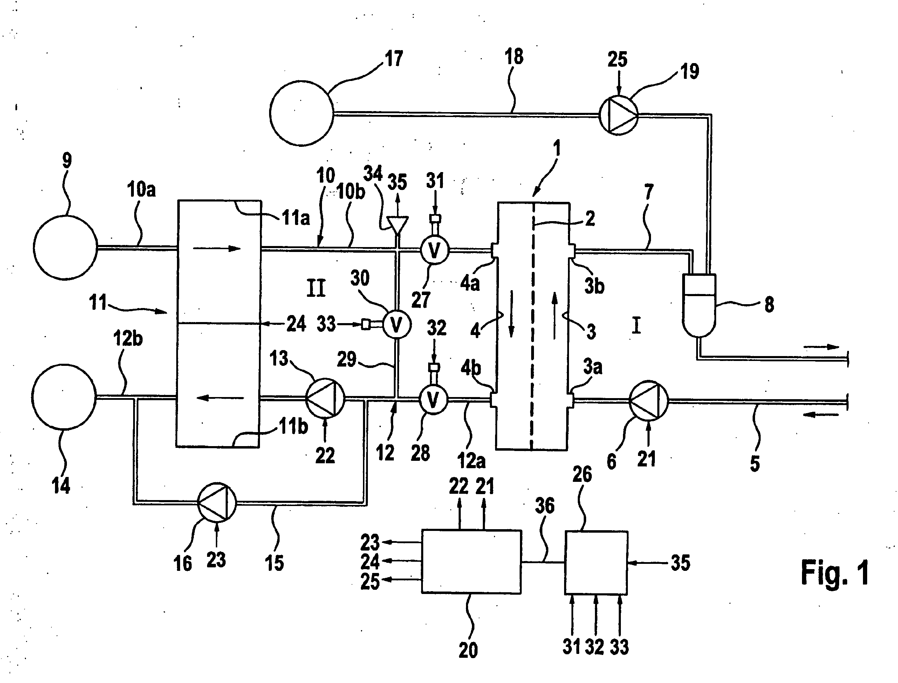

Im folgenden wird ein Ausführungsbeispiel der Erfindung unter Bezugnahme auf die Zeichnung näher erläutert, die in stark vereinfachter schematischer Darstellung die wesentlichen Komponenten einer Hämodiafiltrationsvorrichtung zeigt.

Die Hämodiafiltrationsvorrichtung weist einen Dialysator 1 auf, der durch eine semipermeable Membran 2 in eine Blutkammer 3 und eine Dialysierflüssigkeitskammer 4 getrennt ist. Der Einlass 3a der Blutkammer ist mit einem Ende der arteriellen Blutleitung 5 verbunden, in die eine Blutpumpe 6 geschaltet ist, während der Auslass 3b der Blutkammer mit einem Ende der venösen Blutleitung 7 verbunden ist, in die eine Tropfkammer 8 geschaltet ist. An den anderen Enden der arteriellen und venösen Blutleitung 5,7 befmden sich die nicht dargestellten arteriellen und venösen Kanülen zum Anschluss an den Patienten. Dieser Teil des Flüssigkeitssystems stellt den extrakorporalen Blutkreislauf I der Dialysevorrichtung dar.

Das Dialysierflüssigkeitssystem II der Hämodiafiltrationsvorichtung umfasst eine Einrichtung 9 zur Bereitstellung frischer Dialysierflüssigkeit. Sie ist über einen ersten Abschnitt 10a einer Dialysierflüssigkeitzuführleitung 10 mit dem Einlass der ersten Kammerhälfte 11 a einer Bilanziereinrichtung 11 verbunden. Der zweite Abschnitt 10b der Dialysierflüssigkeitzuführleitung 10 verbindet den Auslass der ersten Bilanzierkammerhälfte 11a mit dem Einlass 4a der Dialysierflüssigkeitskammer 4. Der Auslass 4b der Dialysierflüssigkeitskammer 4 ist über den ersten Abschnitt 12a einer Dialysierflüssigkeitsabführleitung 12 mit dem Einlass der zweiten Bilanzierkammerhälfte 11b der Bilanziereinrichtung 11 verbunden. In den ersten Abschnitt 12a der Dialysierflüssigkeitsabführleitung 12 ist eine Dialysierflüssigkeitspumpe 13 geschaltet. Der Auslass der zweiten Bilanzierkammerhälfte 11b ist über den zweiten Abschnitt 12b der Dialysierflüssigkeitsabführleitung 12 mit einem Auslauf 14 verbunden. Stromauf der Dialysierflüssigkeitspumpe 13 zweigt von der Dialysierflüssigkeitsabführleitung 12 eine Ultrafiltratleitung 15 ab, die ebenfalls zu dem Auslauf 14 führt. In die Ultrafiltratleitung 15 ist eine Ultrafiltrationspumpe 16 geschaltet.

In der Figur sind der besseren Übersichtlichkeit halber nur die beiden Kammerhälften der ersten Bilanzkammer dargestellt. Die Bilanziereinrichtung weist aber noch eine zweite Bilanzkammer auf, die der ersten Kammer parallel geschaltet ist. Darüber hinaus weist die Bilanziereinrichtung ebenfalls nicht dargestellte Ventile zur Steuerung des Dialysierflüssigkeitsflusses auf. Derartige Bilanziersysteme sind beispielsweise in der DE 28 38 414 A1 oder DE 197 08 391 C1 beschrieben.

Die Blutkammer 3 wird von dem Blut des Patienten und die Dialysierflüssigkeitskammer 4 des Dialysators 1 von der Dialysierflüssigkeit durchströmt. Da die Bilanziereinrichtung 11 in den Dialysierflüssigkeitsweg geschaltet ist, kann nur soviel Dialysierflüssigkeit über die Dialysierflüssigkeitszuführleitung 10 zufliessen, wie Dialysierflüssigkeit über die Dialysierflüssigkeitsabführleitung 12 abfliessen kann. Mit der Ultrafiltrationspumpe 16 kann dem extrakorporalen Blutkreislauf I über den Dialysator 1 Flüssigkeit entzogen werden.

Darüber hinaus verfügt die Hämodiafiltrationsvorrichtung über eine Substituatquelle 17, von der eine Substituatleitung 18, in die eine Substituatpumpe 19 geschaltet ist, zu der venösen Tropfkammer 8 führt. Mit der Substituatpumpe 19 kann dem extrakorporalen Blutkreislauf I eine vorgegebene Menge an Substitutionsflüssigkeit aus der Substituatquelle 17 zugeführt werden, während dem Blutkreislauf Flüssigkeit über den Dialysator 1 entzogen wird.

Die Hämodiafiltrationsvorrichtung umfasst eine zentrale Steuer- und Regeleinheit 20, die über Steuerleitungen 21 bis 25 mit der Blutpumpe 6, der Dialysierflüssigkeitspumpe 13, der Ultrafiltrationspumpe 16, der Bilanziereinrichtung 11 und der Substituatpumpe 19 verbunden ist. Die Steuerund Recheneinheit 20 sendet die Steuerbefehle an die einzelnen Komponenten und empfängt von den Komponenten Daten über die Betriebszustände der Komponenten, beispielsweise die Förderraten der Pumpen, die Bilanzierkammertakte, etc.

Zur Überprüfung des Dialysierflüssigkeitsystems II auf Undichtigkeiten weist die Hämodiafiltrationsvorrichtung eine Einrichtung 26 auf, die über eine Datenleitung 27 mit der zentralen Steuer- und Regeleinheit 20 verbunden ist. In die Dialysierflüssigkeitszuführleitung 10 ist stromab der Bilanziereinrichtung 11 ein erstes Absperrventil 27 und in die Dialysierflüssigkeitsabführleitung 12 stromauf der Bilanziereinrichtung 11 ein zweites Absperrventil 28 geschaltet. Stromauf des ersten Absperrventil 27 geht von der Zuführleitung 10 eine Bypassleitung 29 ab, die stromab des zweiten Absperrventils 28 zu der Abführleitung 12 führt. In die Bypassleitung 29 ist ein drittes Absperrventil 30 geschaltet. Die Absperrventile 27, 28, 30 sind elektromagnetisch betätigbare Ventile, die über Steuerleitungen 31, 32, 33 mit der Einrichtung 26 zur Überprüfung des Flüssigkeitssystems verbunden sind. Ferner ist ein Drucksensor 34 vorgesehen, der den Druck in der Dialysierflüssigkeitszuführleitung 10 stromauf des ersten Absperrventils 27 misst. Dieser Drucksensor 34ist über eine Datenleitung 35 ebenfalls mit der Einrichtung 26 verbunden.

Die Überprüfung des Dialysierflüssigkeitssystems II auf Undichtigkeiten erfolgt nach einer Unterbrechung der Dialysebehandlung wie folgt. Die Einrichtung 26 schliesst die Absperrventile 27, 28 und öffnet das Absperrventil 30 für die Dauer des Prüfintervalls Tₚ, so dass die Dialysierflüssigkeitskammer 4 vom Dialysierflüssigkeitssystem II abgetrennt ist. Während des Prüfintervalls Tₚ überwacht die Einrichtung 26 den Betriebsdruck im Dialysierflüssigkeitssystem mit dem Sensor 34 und vergleicht den Betriebsdruck mit einem vorgegebenen Grenzwert. Der Druck steigt während des Prüfintervalls zunächst an, gleicht sich aus und bleibt im Fall eines intakten Systems stabil. Im Fall eines Lecks dagegen fällt er unter den vorgegebenen Grenzwert ab. Dieser Druckabfall ist ein eindeutiger Indikator dafür, dass das UF- Kontrollsystem nicht mehr integer ist. Wenn der Betriebsdruck also den vorgegebenen Grenzwert unterschreitet, erzeugt die Einrichtung 26 ein akkustisches und/oder optisches Alarmsignal. Dieser Druckhaltetest zur Feststellung einer Leckage ist in der DE-A-42 39 937 im Einzelnen beschrieben, auf die ausdrücklich Bezug genommen wird.

Eine Steuereinrichtung 20, die Bestandteil der zentralen Steuer- und Regeleinheit 20 ist, übernimmt vor dem Druckhaltetest die Unterbrechung der Blutbehandlung. Während der Blutbehandlung fördern die Substituatpumpe 19 und die Ultrafiltrationspumpe 16 Dialysierflüssigkeit bzw. Substitutionsflüssigkeit mit einer vorgegebenen Förderrate.

Bei einer Ausführungsform, die aber nicht Gegenstand der Erfindung ist, reduziert die Steuereinrichtung über ein vorgegebenes Zeitintervall T langsam die Förderraten beider Pumpen auf Null.

Erst bei Stillstand der Pumpen aktiviert die Steuereinrichtung 20 die Einrichtung 26 zur Überprüfung des Dialysierflüssigkeitssystems II, die den Dialysator vom Dialysierflüssigkeitssystem abtrennt und den Druckhaltetest durchführt.

Da die Substituat- und Ultrafiltrationspumpe 16, 25 nicht abrupt gestoppt werden, kann sich in dem Leitungsabschnitt der venösen Blutleitung 7 zwischen dem Auslass 3b der Blutkammer 3 und der Tropfkammer 8 nicht eine scharf abgegrenzte Flüssigkeitssäule aus verdicktem Blut bilden. Daher besteht auch nicht die Gefährdung des Patienten durch eine Hämolyse.

Die vorgegebene Zeitdauer T, in der die Förderraten reduziert werden, liegt in der Praxis vorzugsweise zwischen 20 und 30 Sekunden. Die Zeitdauer sollte grösser als 10, vorzugsweise 20 Sekunden sein. Sie ist abhängig von der Blutflussrate und dem Füllvolumen des Dialysators sowie des venösen Schlauchleitungsabschnitts. In der Praxis reicht es aber aus, wenn ein bestimmtes Zeitintervall für alle einstellbaren Flussraten vorgegeben ist. Die Zeitdauer kann abhängig von dem Verhältnis Substitutionsflussrate/Blutflussrate berechnet werden, wobei die Abhängigkeit vorzugsweise linear ist.

Bei der erfindungsgemäßen Vorrichtung arbeitet die Steuereinrichtung wie folgt. Zunächst reduziert die Steuereinrichtung die Ultrafiltrationsrate auf Null, in dem die Ultrafiltrationspumpe 16 abgeschaltet wird. Nach dem Abschalten der Ultrafiltrationspumpe 16 wird die Substituatpumpe 19 noch mit der zuvor eingestellten Förderrate während eines vorgegebenen Zeitintervalls T betrieben, so dass die in der venösen Blutleitung 7 bildende Flüssigkeitssäule aus verdicktem Blut mit der noch zufliessenden Substitutionsflüssigkeit verdünnt wird. Dadurch ist sichergestellt, das verdicktes Blut nicht die venöse Kanüle erreicht. Erst nach dem Stillstand der Substituatpumpe wird die Einrichtung 26 zur Abtrennung des Dialysators und Durchführung des Druckhaltetests aktiviert.

Die vorgegebene Zeitdauer T, in der die Substituatpumpe 19 nach Abschalten der Ultrafiltrationspumpe 16 noch betrieben wird, ist wieder abhängig von dem Füllvolumen des Dialysators 4 und der venösen Blutleitung 7 sowie dem Blutfluss. Die Zeitdauer sollte grösser als 10, vorzugsweise 20 Sekunden sein. In der Praxis liegt das Zeitintervall vorzugsweise zwischen 20 und 30 Sekunden.

## Patentansprüche

1. Vorrichtung zur extrakorporalen Blutbehandlung mit einem extrakorporalen Blutkreislauf (I), der eine erste Kammer (3) eines durch eine semipermeable Membran (2) in die erste Kammer und eine zweite Kammer (4) unterteilten Dialysators (1) oder Filters einschliesst, und einem Flüssigkeitssystem (II), das die zweite Kammer des Dialysators oder Filters einschliesst, wobei dem Blutkreislauf über den Dialysator oder Filter mit einer vorgegebenen Ultrafiltrationsrate Flüssigkeit entzogen und Substitutionsflüssigkeit dem Blutkreislauf stromauf oder stromab des Dialysators oder Filters mit einer Substituatpumpe (19) zugeführt wird, **dadurch gekennzeichnet, dass** zur Unterbrechung der Blutbehandlung eine Steuereinrichtung (20) vorgesehen ist, die derart ausgebildet ist, nach dem Reduzieren der Ultrafiltrationsrate auf einen vorgegebenen Wert die Substituatpumpe (19) mit einer vorgegebenen Förderrate während eines vorgegebenen Zeitintervalls T betrieben wird.

2. Vorrichtung nach Anspruch 1, **dadurch gekennzeichnet, dass** die Ultrafiltrationsrate auf Null reduziert wird.

3. Vorrichtung nach einem der Ansprüche 1 bis 2, **dadurch gekennzeichnet, dass** die Blutbehandlungsvorrichtung eine Dialysierflüssigkeitszuführleitung (10) zum Zuführen von Dialysierflüssigkeit in die Dialysierflüssigkeitskammer (4) und eine Dialysierflüssigkeitsabführleitung (12) zum Abführen von Dialysierflüssigkeit aus der Dialysierflüssigkeitskammer des Dialysators (1) aufweist.

4. Vorrichtung nach Anspruch 3, **dadurch gekennzeichnet, dass** eine Einrichtung (26) zum Überprüfen des Dialysierflüssigkeitssystems (II) auf Undichtigkeiten mittels eines Druckhaltetests in dem Flüssigkeitssystem vorgesehen ist, die zur Abtrennung des Dialysators vom Dialysierflüssigkeitssystem (II) ein erstes Absperrventil (27) in der Dialysierflüssigkeitszuführleitung (10) und ein zweites Absperrventil (28) in der Dialysierflüssigkeitsabführleitung (12) aufweist, wobei die Steuereinrichtung (20) derart ausgebildet ist, dass nach der Unterbrechung der Blutbehandlung die Einrichtung (26) zum Überprüfen des Flüssigkeitssystems auf Undichtigkeiten aktivierbar ist.

5. Vorrichtung nach Anspruch 4, **dadurch gekennzeichnet, dass** eine Bypass-Leitung (29) stromauf des ersten Absperrventils (27) von der Dialysierflüssigkeitszuführleitumg (10) abzweigt, und zu der Dialysierflüssigkeitsabführleitung (12) stromab des zweiten Absperrventils (28) führt, wobei in der Bypass-Leitung ein drittes Absperrventil (30) angeordnet ist, das von der Einrichtung (26) zum Überprüfen des Dialysierflüssigkeitssystems (II) aktivierbar ist.

## Claims

1. Device for extracorporeal blood treatment comprising an extracorporeal blood circuit (I) which comprises a first chamber (3) of a dialyzer (1) or filter divided by a semipermeable membrane (2) into the first chamber and a second chamber (4), and a fluid system (II) which comprises the second chamber of the dialyzer or filter, fluid being removed from the blood circuit via the dialyzer or filter at a predetermined ultrafiltration rate and substitution fluid being fed to the blood circuit upstream or downstream from the dialyzer or filter with a substitute pump (19), **characterized in that**, in order to interrupt the blood treatment, a control unit (20) is provided which is embodied such that, after the reduction of the ultrafiltration rate to a predetermined value, the substitute pump (19) is operated at a predetermined delivery rate for a predetermined time interval T.

2. Device as set forth in claim 1, **characterized in that** the ultrafiltration rate is reduced to zero.

3. Device as set forth in claims 1 to 2, **characterized in that** the blood treatment device has a dialysis fluid feed line (10) for feeding dialysis fluid into the dialysis fluid chamber (4) and a dialysis fluid discharge line (12) for discharging dialysis fluid from the dialysis fluid chamber of the dialyzer (1).

4. Device as set forth in claim 3, **characterized in that** a unit (26) for checking the dialysis fluid system (II) for leaks using a pressure retention test is provided in the system that, to separate the dialyzer from the dialysis fluid system (II), has a first stop valve (27) in the dialysis fluid feed line (10) and a second stop valve (28) in the dialysis fluid discharge line (12), the control unit (20) being embodied such that, after interruption of the blood treatment, the unit (26) for checking
the fluid system for leaks can be activated.

5. Device as set forth in claim 4, **characterized in that** a bypass line (29) branches off from the dialysis fluid feed line (10) upstrfeam from the first stop valve (27) and leads to the dialysis fluid discharge line (12) downstream from the second stop valve (28), a third stop valve (30) being arranged in the bypass line that can be activated by the unit (26) for checking the dialysis fluid system (II).

## Revendications

1. Dispositif permettant le traitement extracorporel du sang, comportant un circuit de circulation sanguine extracorporelle (I) qui contient une première chambre (3) d'un dialyseur (1) ou filtre, divisé par une membrane (2) semi-perméable en ladite première chambre et une deuxième chambre (4), et un système de liquide (II) qui contient la deuxième chambre du dialyseur ou filtre, un liquide étant prélevé dans le circuit de circulation sanguine via le dialyseur ou filtre avec une vitesse d'ultrafiltration prédéfinie et un liquide de substitution étant acheminé dans le circuit de circulation sanguine en amont ou en aval du dialyseur ou filtre au moyen d'une pompe à liquide de substitution (19), **caractérisé en ce que** pour interrompre le traitement du sang, il est prévu un dispositif de commande (20) qui est conçu de telle sorte qu'après la diminution de la vitesse d'ultrafiltration à une valeur prédéfinie, la pompe à liquide de substitution (19) est actionnée avec une vitesse de transport prédéfinie pendant un intervalle de temps T prédéfini.

2. Dispositif selon la revendication 1, **caractérisé en ce que** la vitesse d'ultrafiltration est diminuée à zéro.

3. Dispositif selon l'une quelconque des revendications 1 à 2, **caractérisé en ce que** le dispositif de traitement du sang comporte une ligne d'admission du liquide de dialyse (10) pour acheminer le liquide de dialyse dans la chambre à liquide de dialyse (4), et une ligne d'évacuation du liquide de dialyse (12) pour évacuer le liquide de dialyse hors de la chambre à liquide de dialyse du dialyseur (1).

4. Dispositif selon la revendication 3, **caractérisé en ce qu'**il est prévu une installation (26) permettant de contrôler les inétanchéités dans le système de liquide de dialyse (II) au moyen d'un test de maintien de la pression dans le système de liquide, laquelle installation, pour séparer le dialyseur du système de liquide de dialyse (II), comporte une première vanne d'arrêt (27) dans la ligne d'admission du liquide de dialyse (10) et une deuxième vanne d'arrêt (28) dans la ligne d'évacuation du liquide de dialyse (12), le dispositif de commande (20) étant conçu de telle sorte que, après l'interruption du traitement du sang, ladite installation (26) peut être activée pour contrôler les inétanchéités dans le système de liquide.

5. Dispositif selon la revendication 4, **caractérisé en ce qu'**une ligne à by-pass (29) bifurque de la ligne d'admission du liquide de dialyse (10) en amont de la première vanne d'arrêt (27) et mène vers la ligne d'évacuation du liquide de dialyse (12) en aval de la deuxième vanne d'arrêt (28), une troisième vanne d'arrêt (30) étant montée dans la ligne à by-pass, laquelle vanne peut être activée par l'installation (26) destinée à contrôler le système de liquide de dialyse (II).
